Europäisches Patentamt

European Patent Office

Office européen des brevets

(19)

(11) Publication number: **0 267 795**
**A2**

# EUROPEAN PATENT APPLICATION

(12)

(21) Application number: **87310005.1**

(22) Date of filing: **12.11.87**

(51) Int. Cl.⁴: **C 07 K 13/00**
// C12N15/00, C12P21/02,
A61K37/02

(30) Priority: **14.11.86 US 930768**

(43) Date of publication of application:
**18.05.88   Bulletin   88/20**

(84) Designated Contracting States:
**AT BE CH DE ES FR GB GR IT LI LU NL SE**

(71) Applicant: **Schering Biotech Corporation**
**901 California Avenue**
**Palo Alto California 94304-1104  (US)**

(72) Inventor: **Zurawski, Gerard**
**1028 Wilmington Way**
**Redwood City California 94062  (US)**

**Zurawski, Sandra M.**
**1028 Wilmington Way**
**Redwood City California 94062  (US)**

(74) Representative: **Ritter, Stephen David et ai**
**Mathys & Squire 10 Fleet Street**
**London EC4Y 1AY  (GB)**

(54) Murine-interleukin-2 muteins.

(57)  Mutant murine interleukin-2 proteins (muteins) are pro-
vided. Muteins containing deletions and/or substitutions within
the first 30 N-terminal amino acids are almost all biologically
active. The frequence of biologically active mutants is much
lower for deletions and/or substitutions in other regions of the
native protein.

EP 0 267 795 A2

**Description**

MURINE INTERLEUKIN-2 MUTEINS

This invention relates generally to proteins capable of maintaining in vitro cultures of certain immune system cells, and more particularly, to biologically active mutant proteins (muteins) of the murine lymphokine interleukin-2.

The biological role and importance of human and murine interleukin-2 (IL-2) has been reviewed extensively: e.g. Robb, Immunology Today, Vol. 5, pgs. 203-209 (1984); and Smith, Ann. Rev. Immunol., Vol. 2, pgs. 319-333 (1984). Both human and murine forms of IL-2 have been biochemically characterized, cloned, and sequenced: e.g. Taniguchi et al., Nature, Vol. 302, pgs. 305-310 (1983); Yokota et al., Proc. Natl. Acad. Sci., Vol. 82, pgs. 68-72 (1985); Robb et al., Proc. Natl. Acad. Sci., Vol. 81, pgs. 6486-6490 (1984); Devos et al., Nucleic Acids Research, Vol. 11, pgs. 4307-4323 (1983); Kashima et al., Nature, Vol. 313, pgs. 402-404 (1985); Watson et al., J. Exp. Med., Vol. 150, pgs. 849-861 (1979); and Fuse et al., Nucleic Acids Research, Vol. 12, pgs. 9323-9331 (1984). Muteins of human IL-2 have been constructed using standard genetic engineering techniques: Mark et al., U.S. Patent 4,518,584 dated 21 May 1985; and Wang et al., Science, Vol. 224, pgs. 1431-1433 (1984).

Murine IL-2 is a crucial laboratory reagent for maintaining long-term proliferation of T cells in culture. The availability of alternative sources of this reagent, and the availability of muteins with equivalent or enhanced growth-promoting properties would be highly advantageous to the scientific and industrial research communities.

The present invention is directed to mouse IL-2 muteins defined by Formula I:

```
X(Ala) - X(Pro) - X(Thr) - X(Ser) - X(Ser) - X(Ser) -
                                 10
X(Thr) - X(Ser) - X(Ser) - X(Ser) - X(Thr) - X(Ala) -

X(Glu) - X(Ala) - X(Gln) - X(Gln) - X(Gln) - X(Gln) -
                  20
X(Gln) - X(Gln) - X(Gln) - X(Gln) - X(Gln) - X(Gln) -
                                                30
X(Gln) - X(Gln) - X(His) - X(Leu) - X(Glu) - X(Gln) -

X(Leu) - X(Leu) - X(Met) - X(Asp) - X(Leu) - X(Gln) -
                           40
X(Glu) - X(Leu) - X(Leu) - X(Ser) - X(Arg) - X(Met) -

X(Glu) - X(Asn) - X(Tyr) - X(Arg) - X(Asn) - X(Leu) -
         50
X(Lys) - X(Leu) - X(Pro) - X(Arg) - X(Met) - X(Leu) -
                                                60
X(Thr) - X(Phe) - X(Lys) - X(Phe) - X(Tyr) - X(Leu) -

X(Pro) - X(Lys) - X(Gln) - X(Ala) - X(Thr) - X(Glu) -
                           70
X(Leu) - X(Lys) - X(Asp) - X(Leu) - X(Gln) -   Cys   -

X(Leu) - X(Glu) - X(Asp) - X(Glu) - X(Leu) - X(Gly) -
         80
X(Pro) - X(Leu) - X(Arg) - X(His) - X(Val) - X(Leu) -
                                                90
X(Asp) - X(Leu) - X(Thr) - X(Gln) - X(Ser) - X(Lys) -

X(Ser) - X(Phe) - X(Gln) - X(Leu) - X(Glu) - X(Asp) -
                           100
X(Ala) - X(Glu) - X(Asn) - X(Phe) - X(Ile) - X(Ser) -

X(Asn) - X(Ile) - X(Arg) - X(Val) - X(Thr) - X(Val) -
```

```
                 110
X(Val) - X(Lys) - X(Leu) - X(Lys) - X(Gly) - X(Ser) -
                                                        120
X(Asp) - X(Asn) - X(Thr) - X(Phe) - X(Glu) -    Cys    -

X(Gln) - X(Phe) - X(Asp) - X(Asp) - X(Glu) - X(Ser) -
                            130
X(Ala) - X(Thr) - X(Val) - X(Val) - X(Asp) - X(Phe) -

X(Leu) - X(Arg) - X(Arg) - X(Trp) - X(Ile) - X(Ala) -
          140
X(Phe) - X(Cys) - X(Gln) - X(Ser) - X(Ile) - X(Ile) -

X(Ser) - X(Thr) - X(Ser) - X(Pro) - X(Gln)
```

wherein the term X(Xaa) represents the group of synonymous L-amino acids to the amino acid Xaa, at least one deletion, substitution or insertion as hereinafter defined has been made, and the cysteines at positions 72 and 120 are neither deleted nor substituted. Synonymous amino acids within a group have sufficiently similar physicochemical properties for substitution between members of the group to preserve the biological function of the molecule: Grantham, Science, Vol. 185, pgs. 862-864 (1974). It is clear that insertions and deletions of amino acids may also be made in the above-defined sequence without altering biological function, particularly if only a few amino acids are inserted or deleted, e.g. under five, and amino acids that are critical to a functional conformation are not removed or displaced, e.g. some cysteine residues: Anfisen, "Principles That Govern The Folding of Protein Chains", Science, Vol. 181, pgs. 223-230 (1973). Proteins and muteins produced by such deletions and/or insertions come within the purview of the present invention. Numbers of amino acid residues in the protein of Formula I are in reference to the N-terminus of the protein, and are given above the corresponding amino acids in the Formula. In the discussion following Table I, the letters J and K stand for any two positions in the range 1 to 149 of formula I; except that, for insertions, J can also have the value 0.

Preferably the synonymous amino acid groups are those defined in Table I. More preferably, the synonymous amino acid groups are those before the second slash in each line in Table I; and most preferably the synonymous amino acid groups are those before the first slash in each line in Table I.

## Table I.   Preferred, More Preferred and Most Preferred
### Groups of Synonymous Amino Acids

| Amino Acid | Synonymous Group |
|---|---|
| Ser | Ser,//Thr, Gly, Asn |
| Arg | Arg,/ Lys, His,/Glu, Gln |
| Leu | Leu, Ile, Met,/ Phe,/Tyr, Val |
| Pro | Pro,/Ala,/Thr, Gly |
| Thr | Thr,//Pro, Ser, Ala, Gly, His, Gln |
| Ala | Ala,/Pro,/Gly, Thr |
| Val | Val,/Met, Ile,/Tyr, Phe, Leu |
| Gly | Gly,//Ala, Thr, Pro, Ser |
| Ile | Ile, Met, Leu,/Phe, Val,/Tyr |
| Phe | Phe,/Met, Tyr, Ile, Leu,/Trp, Val |
| Tyr | Tyr,/Phe,/Trp, Met, Ile, Val, Leu |
| Cys | Cys, Ser,//Thr |
| His | His,/Gln, Arg,/Glu, Lys, Thr |
| Gln | Gln,/Glu, His,/Lys, Asn, Thr, Arg |
| Asn | Asn,/Asp,/Ser, Gln |
| Lys | Lys,/Arg,/Glu, Gln, His |
| Asp | Asp,/Asn,/Glu |
| Glu | Glu,/Gln,/Asp, Lys, Asn, His, Arg |
| Met | Met, Ile, Leu,/Phe, Val/ |

The invention includes the polypeptides of Formula I with amino acid substitutions (of a synonymous amino acid for an amino acid of the native mouse IL-2) at a single position or at multiple positions. The term "N-fold substituted (J-K)" is used to describe a subset of polypeptides defined by Formula I wherein the native amino acids have been substituted at amino acids J through K, inclusive, by synonymous amino acids at 1 to N positions. Thus, for example, the group of 1-fold substituted (1-10) polypeptides of Formula I consists of 36 polypeptides for the preferred groups of synonymous amino acids, 2 for the more preferred groups of synonymous amino acids, and none for the most preferred groups of synonymous amino acids. These numbers were obtained by taking the numbers of amino acids in the various synonymous groups, summing these, and subtracting 10 from the result to allow for those 10 amino acid residues present at positions 1-10.

Likewise, the term "N-fold inserted (J-K)" in reference to the polypeptides of Formula I is used to describe a set of polypeptides wherein from 1 to N amino acids have been inserted within the polypeptide segment between amino acids J and K. Note that here J can take the value 0. Preferably, the inserted amino acids are selected from the preferred groups of synonymous amino acids of the amino acids flanking the insertion; more preferably they are selected from the more preferred groups of synonymous amino acids of the amino acids flanking the insertion, and most preferably they are selected from the most preferred groups of synonymous amino acids of the amino acids flanking the insertion. These groups are all indicated in Table I. Thus, for example, one subgroup of the group of 1-fold inserted (1-11) peptides comprises a peptide of formula I having an amino acid inserted between the penultimate N-terminal X(Pro) and the adjacent X(Thr). The insertions defining the members of this subgroup are preferably selected from Pro, Ala, Gly, Thr, Ser, Gln, Glu, and His; more preferably they are selected from Ala, Pro, and Thr, and most preferably they are selected from Pro and Thr. Insertions can be made between any adjacent amino acids with the sequence of amino acids 1 through 11. Since there are 10 possible insertion locations, and since multiple insertions can be made at the same location, a 2-fold inserted (1-11) polypeptide of Formula I gives rise to 10x10 = 100 subgroups of polypeptides, and the size of each subgroup depends on the sizes of the synonymous amino acid groups of the amino acids flanking the insertions, and of the rest of the amino acids in the polypeptide.

The term "N-fold deleted (J-K)" in reference to the polypeptides of Formula I is used to describe a set of polypeptides having from 1 to N amino acids deleted from the sequence of amino acids at the positions J through K, inclusive. Thus, the set of 1-fold deleted (1-10) polypeptides of Formula I consists of 149 subgroups of polypeptides each 148 amino acids in length (148-mers). Each of the subgroups in turn consists of all the 148-mers defined by the preferred, more preferred, and most preferred synonymous amino acid groups.

In defining the muteins of the invention, whenever an amino acid sequence is subject to substitutions, deletions, and insertions, deletions are made first, substitutions are made second up to the lesser of the number of amino acids remaining after deletions are made and the multiplicity of the substitutions. and insertions are made last.

Preferably, muteins of the invention include 13-fold deleted (1-13), 12-fold deleted (14-25), 1-fold deleted (26-140), 5-fold deleted (141-149), 5-fold substituted (1-13), 12-fold substituted (14-25), 5-fold substituted (26-38), 5-fold substituted (39-140), 5-fold substituted (141-149), 5-fold inserted (0-13), 1-fold inserted (14-149) polypeptides of Formula I wherein the cysteines at positions 72 and 120 ($Cys^{72}$ and $Cys^{120}$, respectively) are neither substituted nor deleted.

More preferably, muteins of the invention include 12-fold deleted (14-25), 1-fold deleted (26-140), 5-fold deleted (141-149), 5-fold substituted (1-25), 3-fold substituted (26-38), 3-fold substituted (39-140), 5-fold substituted (141-149), 5-fold inserted (0-13), 1-fold inserted (14-149) polypeptides of Formula I wherein $Cys^{72}$ and $Cys^{120}$ are neither substituted nor deleted.

Most preferably, muteins of the invention include 12-fold deleted (14-25), 5-fold deleted (141-149), 3-fold substituted (1-25), 2-fold substituted (26-38), 3-fold substituted (141-149) polypeptides of formula I wherein $Cys^{72}$ and $Cys^{120}$ are neither substituted nor deleted.

Throughout, standard abbreviations are used to designate amino acids, nucleotides, restriction endonucleases, and the like: e.g. Cohn, "Nomenclature and Symbolism of α-Amino Acids", Methods in Enzymology, Vol. 106, pgs. 3-17 (1984); Wood et al., Biochemistry: A Problems Approach, 2nd ed. (Benjamin, Menlo Park, 1981); and Roberts, "Directory of Restriction Endonucleases", Methods in Enzymology, Vol. 68, pgs. 27-40 (1979).

In the accompanying drawings:

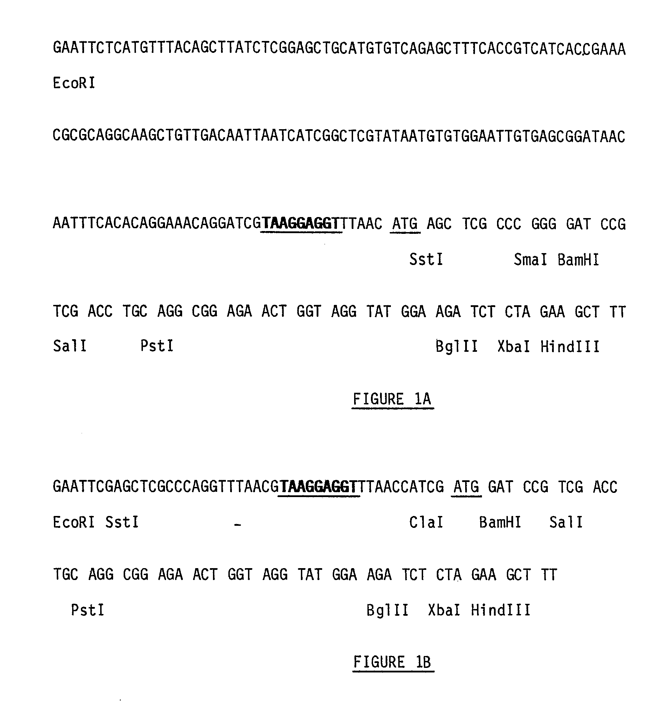

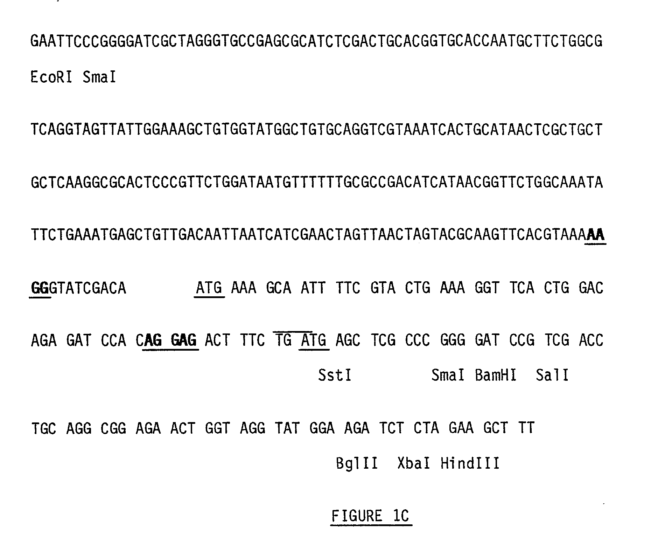

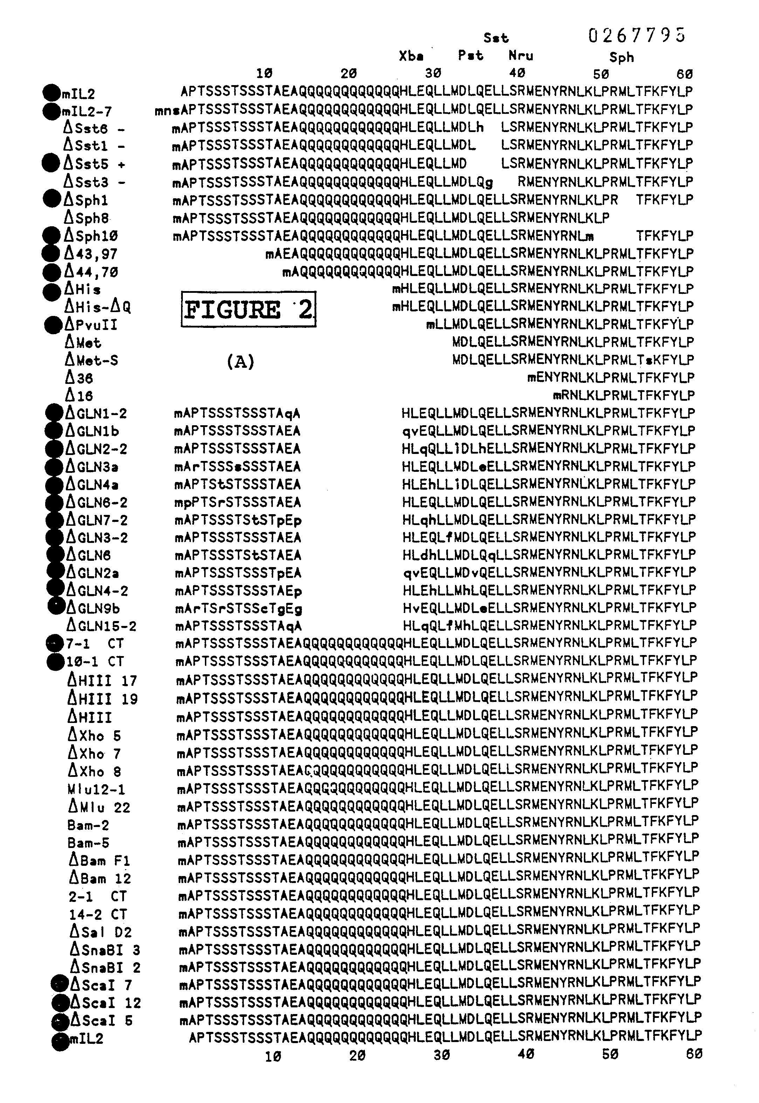

Figures 1A-C illustrate nucleotide sequences adjacent to the initiator ATG codon in three E. coli expression vector plasmids (TAC-RBS: Figure 1A; TRP C11: Figure 1B; and TCV: Figure 1C). The sequences commence at an EcoRI restriction site and end at a HindIII site. Arrows indicate the 5' nucleotide after restriction by various nucleases which are indicated under the arrows. Ribosome binding sequences showing complementarity to the 3' end of 16s ribosomal RNA are shown in bold type and are underlined. ATG initiator codons are underlined and the E. coli trpLE peptide termination codon is indicated by a superscript line (Fig. 1C).

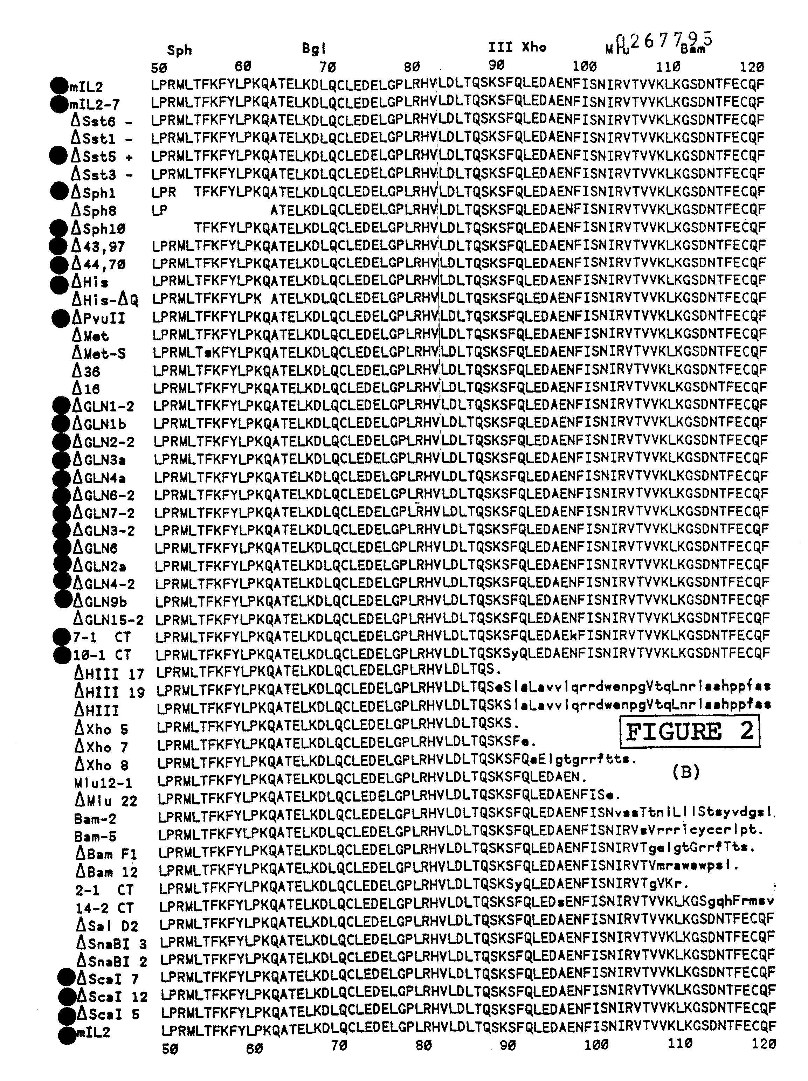

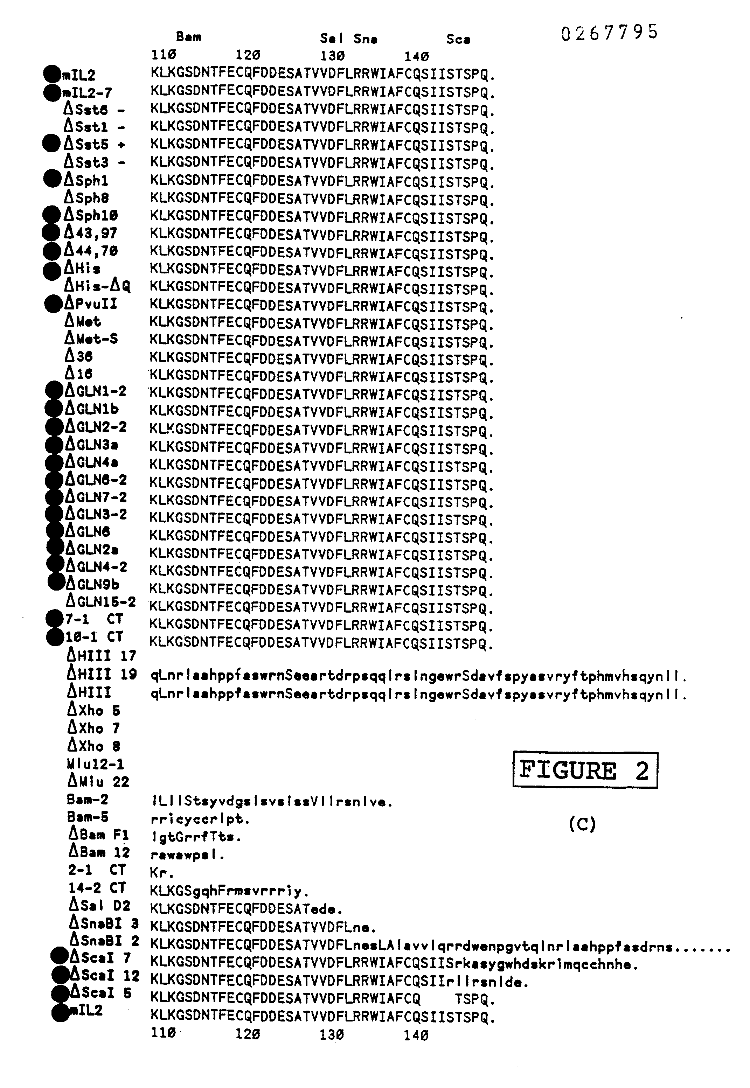

Figure 2 (spread over three sheets A, B and C, with overlaps) shows the amino acid sequences of exemplary muteins of the invention relative to native mouse IL-2. Standard notations for amino acids are used (as in "Nomenclature and Symbolism of α-Amino Acids" by Waldo E. Cohn, Methods in Emzymology, Vol. 106 (1984), pp. 3-17), and lower case letters indicate substitutions or insertions relative to the native sequence. A black circle to the left of the mutant protein's symbol indicates that the mutant protein is biologically active. Positions of unique restriction endonuclease sites for cassette mutagenesis are indicated at the top of the figure.

Once nucleic acid sequence information and/or amino acid sequence information is available for a native protein, a variety of techniques become available for producing virtually any mutation in the native sequence. Shortle, in Science, Vol. 229, pgs. 1193-1201 (1985), reviews techniques for mutating nucleic acids which are applicable to the present invention. Preferably, mutants of the native mouse IL-2 are produced by site-specific oligonucleotide-directed mutagenesis: e.g. Zoller and Smith, Methods in Enzymology, Vol. 100, pgs. 468-500 (1983); Mark et al., U.S. Patent 4,518,584 entitled "Human Recombinant Interleukin-2 Muteins" or by so-called "cassette" mutagenesis described by Wells et al. in Gene, Vol. 34, pgs. 315-323 (1985), by Estell et al. in Science, Vol. 233, pgs. 659-663 (1986), and also essentially by Mullenbach et al., J. Biol. Chem., Vol. 261, pgs. 719-722 (1986), and by Feretti et al., Proc. Natl. Acad. Sci., Vol. 83, pgs. 597-603 (1986). In sections below, the notation used by Estell et al. (cited above) to identify muteins is followed and generalized. For example, "mouse IL-2 mutein $Leu^{82}$" (or simply "$Leu^{82}$" if the native protein is understood from the context) indicates a polypeptide whose amino acid sequence is identical to that of the native protein except for position 82 with respect to the N-terminus. At that position Leu has been substituted for His. Where the mutein contains more than one substitution, e.g. Leu for His at position 82, and Asp for Leu at position 111, the mutein is referred to as mouse IL-2 mutein ($Leu^{82}$, $Asp^{111}$). Deletions are indicated by "Δ's". For example, a mutein lacking Gln at position 71 is referred to as mouse IL-2 mutein $\Delta^{71}$. Insertions are indicated by "IN(Xaa)'s". For example, a mutein with a Leu inserted after Gln at position 71 is referred to as mouse IL-2 mutein $IN^{71}$(Leu). Thus, mouse IL-2 mutein ($Ser^{13}$, $\Delta^{71}$, $IN^{94}$(Gly)) represents the native mouse IL-2 sequence which has been modified by replacing Glu by Ser at position 13, deleting Gln at position 71, and inserting Gly immediately after Leu at position 94. Insertion of multiple amino acids at the same site is indicated by $IN^{i}$($Xaa_1$-$Xaa_2$-$Xaa_3$-...), where $Xaa_1$-$Xaa_2$-$Xaa_3$... is the sequence inserted after position i. N-terminal additions are indicated by superscript "0", e.g. IS⁰(Xaa), and a sequence of deletions, for example of amino acids 6-10, is designated either as $\Delta^{6-10}$ or as $(\Delta^6, \Delta^7, \Delta^8, \Delta^9, \Delta^{10})$.

Most preferably cassette mutagenesis is employed to generate mouse IL-2 muteins. As described more fully below, a synthetic mouse IL-2 gene has been constructed with a sequence of restriction endonuclease sites that are unique when inserted in a appropriate vector and are spaced approximately uniformly along the gene. The unique restriction sites allow segments of the gene to be conveniently excised and replaced with synthetic oligonucleotides (ie. "cassettes") which code for desired muteins.

Determination of the number and distribution of unique restriction sites entails the consideration of several factors including (1) preexisting restriction sites in the vector to be employed in expression, (2) whether species-specific or genera-specific codon usage is desired, and (3) the convenience and reliability of synthesizing and/or sequencing the segments between the unique restriction sites.

## I. Bacterial Strains, Expression Vectors, and Recombinant DNA Protocols

Cloning and expression are carried out in standard bacterial systems, for example E. coli K-12 strain JM101 described by Vieira and Messing, Gene, Vol. 19, pgs. 259-268 (1982), which is commercially available. Expression vectors were purchased commercially (e.g. pUC18 from Pharmacia) or were constructed from commercially available vectors.

Restriction endonuclease digestions, and DNA polymerase, kinase, ligase and exonuclease reactions were performed using standard techniques, e.g. Maniatis et al., Molecular Cloning: A Laboratory Manual (Cold Spring Harbor Laboratory, New York, 1982). Enzymes are available from commercial sources, e.g. New England Biolabs or Boehringer Mannheim.

The alkaline method (Maniatis et al., cited above) was used for small scale plasmid preparations. For large scale preparations a modification of the alkaline method was used in which an equal volume of isopropanol was used to precipitate nucleic acids from the cleared lysate. Precipitation with cold 2.5 M ammonium acetate was used to remove RNA prior to mixing with ethidium bromide and separation by equilibrium density centrifugation in cesium bromide.

For filter hybridizations Whatman 540 filter circles were used to lift colonies which were then lysed and fixed by successive treatments with 0.5M NaOH, 1.5M NaCl; 1M Tris.HCl pH 8.0, 1.5M NaCl (2 min each); and heating at 80°C (30 min). Hybridizations were in 6xSSPE, 20% formamide, 0.1% sodium dodecylsulphate (SDS), 100 µg/ml E. coli tRNA, 100 µg/ml Coomassie Brilliant Blue G-250 (Biorad) at 42°C for 6 hrs using ³²P-labelled (kinased) synthetic DNAs. (20xSSPE is prepared by dissolving 174 g of NaCl, 27.6 g of $NaH_2PO_4\bullet H_2O$, and 7.4 g of EDTA in 800 ml of $H_2O$. The pH is adjusted to 7.4 with NaOH, the volume is adjusted to 1 liter, and the solution is sterilized by autoclaving.)

Filters were washed twice (15 min, room temperature) with 1xSSPE, 0.1% SDS. After autoradiography (Fuji RX film), positive colonies were located by aligning the regrown colonies with the blue-stained colonies on the filters.

DNA was sequenced by either the chemical degradation method of Maxam and Gilbert, Methods in Enzymology, Vol. 65, pg. 499 (1980), or by the dideoxy method of Sanger et al., Proc. Natl. Acad. Sci., Vol. 74, pg. 5463 (1977). Templates for the dideoxy reactions were either single stranded DNAs of relevant regions recloned into M13mp vectors, e.g. Messing et al., Nucleic Acids Res., Vol. 9, pg 309 (1981), or double-stranded DNA prepared by the minialkaline method and denatured with 0.2M NaOH (5 min, room temperature) and precipitated from 0.2M NaOH, 1.43M ammonium acetate by the addition of 2 volumes of ethanol. Dideoxy reactions were done at 42°C.

The TAC-RBS vector was constructed by filling-in with DNA polymerase the single BamHI site of the tacP-bearing plasmid pDR540 (Pharmacia, PL). This was then ligated to unphosphorylated synthetic oligonucleotides (Pharmacia, PL) which form a double-stranded fragment encoding a consensus ribosome binding site (RBS, GTAAGGAGGTTTAAC). After ligation, the mixture was phosphorylated and religated with the SstI linker ATGAGCTCAT. This complex was then cleaved with SstI and EcoRI, and the 175 bp fragment isolated by polyacrylamide gel electrophoresis (PAGE) and cloned into EcoRI-SstI-restricted pUC12 (Pharmacia, PL). One clone with the correct sequence was selected and the 180 bp EcoRI-BamHI tacP-RBS fragment was recloned into pMT11hc. pMT11hc is a small (2.3 kb), high copy, AMPR, TETS derivative of pBR322 that bears the πVX plasmid EcoRI-HindIII polylinker region. The sequence of the RBS-ATG-polylinker regions of the final construction (called TAC-RBS) is shown in Figure 1A.

The TRPC11 vector was constructed by ligating the synthetic consensus RBS fragment to ClaI linkers (ATGCAT) and by cloning the resulting fragments into ClaI-restricted pMT11hcClaI. This plasmid was constructed by restricting pMT11hc with EcoRI and BamHI, filling in and ligating with ClaI linker (CATCGATG), thereby restoring the EcoRI and BamHI sites and replacing the SmaI site with a ClaI site. One transformant from the TRPC11 construction had a tandem RBS sequence flanked by ClaI sites. One of the ClaI sites and part of the second copy of the RBS sequence were removed by digesting this plasmid with PstI, treating with Bal31 nuclease, restricting with EcoRI and treating with T4 DNA polymerase in the presence of all four deoxynucleotide triphosphates. The resulting 30-40 bp fragments were recovered by PAGE and cloned into SmaI restricted pUC12. A 248 bp E. coli trpP-bearing EcoRI fragment derived from pKC101 (Nichols, B.P. and C. Yanofski: Plasmids containing the trp promoters of Escherichia coli and Serratia marcescens and their use in expressing cloned genes, Methods Enzymol., 1983, 101:155) was then cloned into the EcoRI site to complete the TRPC11 construction.

6

The TCV vector was constructed by cloning the 330 bp Sau3A-BglII trpLE1417-bearing fragment of pVVI (Nichols et al., cited above) into BamHI- and BglII-restricted pMT11hc. A clone with an intact BglII site was restricted with BglII and ligated to a synthetic double-stranded DNA fragment having a protruding GATC end, a flush end, and a sequence of GATCCACAGGAGACTTTCTGATGAGCTC. The resulting ligation was restricted with EcoRI and the mixture was cloned into EcoRI- and Sst1-restricted pUC18 (Pharmacia) DNA. The EcoRI-BamHI trpP-TrpLE-ATG-bearing fragment was then recloned into pMT11hc restricted with EcoRI and BamHI. The structure and sequence of the trpLE-ATG region of the TCV construction is shown in Figure 1C.

Construction of murine IL-2 (mIL-2) expression plasmids.

TAC7 mIL-2 was constructed by ligating Sst1-restricted, T4-DNA-polymerase-treated and BamHI-restricted TAC-RBS vector DNA with the 756 bp HincII-BamHI fragment from pMT-18 mIL-2 cDNA (Yokota, T., N. Arai, F. Lee, D. Rennick, T.R. Mosmann, and K. Arai: Use of a cDNA expression vector for isolation of mouse interleukin 2 cDNA clones: Expression of T-cell growth-factor activity after transfection of monkey cells; Prod. Natl. Acad. Sci. U.S.A., 1985, 82:68). Note that the mIL-2 coding region in this construction has an additional two amino acids (Asn,Ser) derived from the leader sequence encoded by the mIL-2 cDNA.

The exTAC mIL-2 plasmid (ex denotes that the f-MET-initiated region of mIL-2 is exactly that of the mature processed protein) was constructed as above, except that the mIL-2 fragment used was a 750 HinPI-BamHI fragment in which the HinPI site had been filled with T4 DNA polymerase and dCTP and then treated with mung bean nuclease.

The exTCV mIL-2 plasmid was constructed as above, except that TCV DNA was used in place of TAC-RBS. The exTRP mIL-2 plasmid was constructed by first inserting the sequence ATCGATG between bsp 108 and 109 in the mIL-2 cDNA sequence of Yokota et al. (cited above). This insertion was performed using site-directed mutagenesis. The 756 bp ClaI-BamHI mIL-2 fragment from the insertion mutant was then cloned into ClaI- and BamHI-restricted TRPC11 DNA.

The TRP5 mIL-2 plasmid was constructed as described for TAC7 mIL-2, except that the vector DNA was a derivative of the TCV vector in which the trp LE peptide is in phase with the ATG codon proximal to the SstI site.

The TRP mIL-2 Bal31 deletion plasmids were constructed by restricting exTRP mIL-2 DNA with ClaI, recessing with Bal31, treating with DNA polymerase, ligating with kinased ClaI linker DNA (CATCGATG) and restricting with BamHI. PAGE-purified ClaI-BamHI DNA (750 bp-600 bp) was ligated to BamHI- and ClaI-restricted TRPC11 DNA.

The TRP mIL-2 ΔGLN plasmids were constructed by ligating a synthetic ClaI-SstI fragment to ClaI- and Sst-restricted exTRPC11 mIL-2 DNA. The synthetic DNA encoded the ClaI-SstI region of exTRPC11 mIL-2 (SstI is at mIL-2 bp 196-201: Yokota et al. (cited above)), except that the poly-Gln-coding region (mIL-2 bp 150-186: Yokota et al. (cited above)) was absent. Plasmids failing to hybridize to kinased poly-CAG DNA were tested for their ability to direct the synthesis of a smaller form of mIL-2 protein as described below. The synthetic-derived part of plasmids passing that test was then sequenced using a synthetic mIL-2 primer corresponding to bps 273-254 (see Yokota et al., cited above).

## II. DNA Syntheses And Cloning

DNA was synthesized by phosphoramidite chemistry using Applied Biosystems 380A synthesizers. Synthesis, deprotection, cleavage and purification (7M urea PAGE, elution, DEAE-cellulose chromatography) were done as described in the 380A synthesizer manual. Complementary strands of synthetic DNAs to be cloned (400ng each) were mixed and phosphorylated with polynucleotide kinase in a reaction volume of 50 μl. This DNA was ligated with 1 μg of vector DNA digested with appropriate restriction enzymes, and ligations were in a volume of 50 μl at room temperature for 4 to 12 hours. Conditions for phosphorylation, restriction-enzyme digestions, polymerase reactions, and ligation have been described (Maniatis et al., cited above). Colonies were scored for lacZ+ by plating on L agar supplemented with ampicillin, isopropyl-1-thio-beta-D-galactoside (IPTG) (0.4 mM) and 5-bromo-4-chloro-3-indolyl-beta-D-galactopyranoside (x-gal) (40 μg/ml). When natural DNA fragments were used in clonings, 20 μg of plasmid was digested and the products separated by preparative 6% PAGE (Maxam and Gilbert, cited above) and further purified by DEAE-cellulose chromatography (Maniatis et al., cited above). One-tenth of the fragment was ligated with 1 μg of vector DNA digested with appropriate restriction enzymes.

## III. Preparation of Protein Extracts and Bioassays of Mouse IL-2 Activity

Single colonies were picked into 0.2 ml of L broth (described in Maniatis et al., cited above) with 100 μg/ml ampicillin and grown at 37°C overnight without shaking. A further 2 mls of medium was added and the cells were grown with shaking at 37°C for 6 hrs. Cells haboring the TAC-derived plasmids were induced by the addition of 0.4 mM IPTG (Sigma) after 2 hrs. of growth. Cells were harvested by centrifugation (13,000 rmp, 2 min, Biofuge A), resuspended in 1 ml of phosphate-buffered saline (PBS, Sigma) with 1mM phenylmethylsulfonyl chloride (PMSF) (Sigma) and sonicated for 40 pulses (50%) at 40 watts using the miniprobe of the Branson Cell Disruptor 200. Following the addition of 100 μl of 10% SDS (Biorad) and incubation at 37°C for 15 min, the samples were centrifuged (13,000 rpm, 15 min, Biofuge A). Dilutions of the samples were bioassayed by several available methods.

Several important standard assays have been described for TCGF activity, e.g. Devos et al., Nucleic Acids Research, Vol. 11, pgs. 4307-4323 (1983); Thurman et al., J. Biol. Response Modifiers, Vol. 5, pgs 85-107 (1986); and Robert-Guroff et al., Chapter 9 in Guroff, Ed. Growth and Maturation Factors (John Wiley, New York, 1984). Generally, the TCGF assays are based on the ability of a factor to promote the proliferation of peripheral T lymphocytes or IL-2 dependent T cell lines: e.g., Gillis et al., J. Immunol., Vol. 120, pg. 2027 (1978) who describe the IL-2 dependent cell line CTLL-2 available from the American Type Culture Collection (Rockville, MD) under accession number TIB 214. Proliferation can be determined by standard techniques, e.g. tritiated thymidine incorporation, or by colorimetric methods: Mosmann, J. Immunol. Meth., Vol. 65, pgs. 55-63 (1983).

IL-2 was assayed on the murine T cell line HT2, described by Watson in J. Exp. Med., Vol. 150, pg. 1510 (1979), using the MTT colorimetric proliferation assay of Mosmann (cited above). Samples were assayed without filtration at a starting dilution of 1/40. The assay contained 50 μg/ml gentamycin (Sigma) to prevent growth of E. coli cells that survived the extraction procedure. One unit of IL-2 is defined as the amount of factor that, in 0.1 ml, results in a signal in the MTT assay equal to 50% of the maximum signal.

## IV. Experimental

The coding region corresponding to the mature mIL-2 protein was inserted immediately distal to the promoter-RBS-ATG regions of each of three expression plasmid vectors, TAC-RBS, TRPC11 and TCV. These vectors were designed as general vehicles for the expression of heterologous genes in E. coli. They have in common high copy number, strong promoters (tacP and trpP), and sequences distal to the translational initiation site with unique sites for many restriction nucleases to facilitate insertion of cDNA sequences. They differ in the nature of their ribosome binding sites in that, although TAC-RBS and TRPC11 have identical RBS sequences, they vary in spacing and sequence between the RBS and ATG sequences. The TCV RBS is designed to be translationally coupled to trpLE peptide translation. Plasmids bearing mIL-2 coding sequences in these three expression vectors were tested for their abilities to direct the synthesis of the mIL-2 protein in the E. coli host JM101. Total E. coli proteins were analyzed by SDS-PAGE with subsequent Western blotting using anti-IL-2 monoclonal DMS-1 (Table II) (a monoclonal antibody specific for mouse IL-2 and available from Genzyme), or the like; e.g. the monoclonal disclosed by Gillis in U.S. Patent 4,411,993 or that produced by hybridoma 7D4 or 24B6 available from the American Type Culture Collection under accession number CRL 1698 or HB8794, respectively. The expected 16kd mIL-2 polypeptide was detected using all three vector systems. The TAC ex mIL-2 plasmid consistently produced the greatest amount of mIL-2 protein. The expressed mIL-2 protein partitioned with the membrane fraction after lysis of the cells, and analysis of 1% SDS-extracted proteins from this fraction by SDS-PAGE revealed that up to 20% of the protein was mIL-2. The SDS-extracted material was highly active in sustaining the proliferation of T-cells and has proved suitable for the long term maintenance of such cells.

## mIL-2 proteins with additional N-terminal amino acids are active.

To analyze the role of the N-terminal region in the function of mIL-2 protein, the effects of two N-terminal addition mutations upon the activity of mIL-2 were evaluated. Two plasmids that direct the synthesis of N-terminal addition mIL-2 proteins were constructed. One such plasmid, TAC7 mIL-2, encodes an mIL-2 protein with an additional N-terminal Asn-Ser. The other plasmid, TRP5 mIL-2, encodes an mIL-2 protein with an additional 20 N-terminal amino acids derived from the E. coli trpLE peptide-coding region of the TCV vector. These plasmids directed abundant synthesis of each mutant mIL-2 protein in E. coli. Since the absolute yield of mIL-2 protein varied between plasmids, Western blot analysis was utilized with the DMS-1 monoclonal antibody to assess the approximate levels of mIL-2 protein present in samples which were also assayed for T-cell proliferation activity. This procedure allowed estimation of the relative specific activities of the various mutant mIL-2 proteins described, on the assumption that the DMS-1 monoclonal antibody was equally reactive with the various mIL-2 proteins.

TABLE II

Activity of IL-2 expressed from various vectors

| Mutant protein | DMS-1[a] | Units x $10^{-5}$ | Relative[b] activity |
|---|---|---|---|
| TAC ex mIL-2 | 4.54 ± 0.22 | 7.58 ± 1.38 | 100 |
| TRP ex mIL-2 | 5.12 ± 0.13 | 8.97 ± 0.39 | 105 |
| TCV ex mIL-2 | 1.19 ± 0.07 | 2.43 ± 0.19 | 122 |
| TAC7 mIL-2 | 6.68 ± 0.18 | 11.93 ± 1.48 | 107 |
| TRP5 mIL-2 | 6.65 ± 0.23 | 3.89 ± 0.15 | 35 |

a    Values are arbitrary units of area.

b    Ratio of biological activity to immunoreactivity, normalized to a value of 100 for TAC ex mIL-2

TABLE III

Activity and immunoreactivity of mIL-2 mutants

| Mutant protein | DMS-1[b] | Activity[c] Units/ml x $10^{-3}$ | Relative activity | S4B6[d] |
|---|---|---|---|---|
| TAC ex mIL-2 | 15.4 | 951 | 100 | + |
| TRP ex mIL-2 | 16.4 | 591 | 67 | + |
| Δ97 | 11.2 | 437 | 62 | + |
| Δ70 | 12.1 | 397 | 50 | + |
| ΔPvuII | 9.0 | 2 | 0.4 | − |
| Δ36 | 6.0 | − | − | − |
| Δ16 | n.d. | − | − | − |
| ΔGln1-2[a] | 0.3 | 6 | 36 | + |
| ΔGln1b | 16.3 | 284 | 28 | + |
| ΔGln2-2 | 16.3 | 184 | 18 | + |
| ΔGln3a | 11.7 | 232 | 32 | + |
| ΔGln4a | 4.4 | 49 | 18 | + |
| ΔGln6-2 | 6.9 | 93 | 22 | + |
| ΔGln7-2 | 16.9 | 164 | 18 | + |
| ΔGln3-2 | 10.8 | 37 | 5.5 | + |
| ΔGln6 | 20.6 | 120 | 9.4 | + |
| ΔGln2a | 15.0 | 6 | 0.7 | − |
| ΔGln4-2 | 2.3 | 2 | 1.6 | + |
| ΔGln9b | 10.4 | 10 | 1.5 | + |
| ΔGln15-2 | 1.7 | − | − | − |

a    This mutant protein was assayed separately using TRP ex mIL-2 as the standard.

b    Values are arbitrary of area. n.d. indicates that no peak was detected by the densitometer.

c    Under the conditions of the assay $0.2 \times 10^{-3}$ units/ml is the minimal value detectable.

d    Immunoreactivity was determined by protein blot analysis as described in Materials and Methods. Proteins that were not detectably recognized by S4B6 antibody or that were greatly (>10-fold) reduced in S4B6 immunoreactivity are classified as −.

## TABLE IV

### Relative activity of mIL-2 mutant proteins
### on mouse and human T cells.

| IL-2 protein | Activity[a] (Mouse) | Activity[a] (Human) | Ratio (m/h)[b] |
|---|---|---|---|
| ex TAC mIL-2 | 507 | 17.9 | 29.8 ± 4.8 |
| | 129 | 5.0 | |
| | 713 | 20.3 | |
| ΔGln3a | 277 | 2.9 | 85.3 ± 19.4 |
| | 339 | 3.4 | |
| | 157 | 2.5 | |
| ΔGln4a | 96 | 11.9 | 7.0 ± 1.0 |
| | 76 | 11.6 | |
| | 55 | 8.9 | |
| human IL-2 | 2.0 | 1.6 | 1.3 ± 0.1 |
| | 1.8 | 1.4 | |
| | 2.2 | 1.5 | |

a   Units/ml T cell growth factor activity, x $10^{-3}$; results from three replicate bacterial cultures are shown.

b   The ratio of mouse to human units was calculated for each replicate culture, and then the means and standard deviations were determined.

Table II shows that this procedure is consistent and reproducible. Table II also shows that an additional Asn-Ser at the N-terminus of mIL-2 did not significantly affect the specific activity of the protein. However, an additional 20 amino acids in the TRP5 mIL-2 protein resulted in a modest reduction in specific activity, although, given sufficient quantities, this mutant protein was fully active in sustaining the growth of T-cells.

N-terminal mIL-2 deletions identify two functionally distinct regions.

A series of N-terminal deletion derivatives of the TRP exIL-2 expression plasmid was constructed. Figure 2 shows the extent of the various N-terminal deletions that were analyzed. Deletion of the N-terminal 11, 12 or 13 amino acids had no significant effect on specific activity of the expressed protein (Fig. 2). However, deletion proteins lacking the N-terminal 41 or 45 amino acids were completely inactive (Fig. 2). Deletion of the N-terminal 30 amino acids resulted in a drastic decrease in activity (Fig. 2), although this protein (ΔPvuII) did have slight residual activity.

A series of muteins were constructed which contained deletions of the entire poly-Gln region from positions 15 to 26, inclusive (the "ΔGln" mutants). They were constructed by replacing native N-terminal coding regions with synthetic DNA (coding for the mutein) in the TRP ex mIL-2 plasmid.

The descriptions of the foregoing embodiments of the invention have been presented for purpose of illustration and description. They are not intended to be exhaustive or to limit the invention to the precise forms disclosed, and obviously many modifications and variations are possible in light of the above teaching. The embodiments were chosen and described in order to best explain the principles of the invention and its practical application to thereby enable others skilled in the art to utilize the invention in various embodiments and with various modifications as are suited to the particular use contemplated.

**Claims**

1. Murine Interleukin-2 muteins comprising polypeptide sequences selected from the group consisting of 13-fold deleted (1-13), 12-fold deleted (12-25), 1-fold deleted (26-140), 5-fold deleted (141-149), 5-fold substituted (1-13), 12-fold substituted (14-25), 5-fold substituted (26-38), 5-fold substituted (39-140), 5-fold substituted (141-149), 5-fold inserted (0-13), 1-fold inserted (14-149) polypeptides of the formula:

```
X(Ala) - X(Pro) - X(Thr) - X(Ser) - X(Ser) - X(Ser) -
                                10
X(Thr) - X(Ser) - X(Ser) - X(Ser) - X(Thr) - X(Ala) -

X(Glu) - X(Ala) - X(Gln) - X(Gln) - X(Gln) - X(Gln) -
            20
X(Gln) - X(Gln) - X(Gln) - X(Gln) - X(Gln) - X(Gln) -
                                                30
X(Gln) - X(Gln) - X(His) - X(Leu) - X(Glu) - X(Gln) -

X(Leu) - X(Leu) - X(Met) - X(Asp) - X(Leu) - X(Gln) -
                              40
X(Glu) - X(Leu) - X(Leu) - X(Ser) - X(Arg) - X(Met) -

X(Glu) - X(Asn) - X(Tyr) - X(Arg) - X(Asn) - X(Leu) -
            50
X(Lys) - X(Leu) - X(Pro) - X(Arg) - X(Met) - X(Leu) -
                                                60
X(Thr) - X(Phe) - X(Lys) - X(Phe) - X(Tyr) - X(Leu) -

X(Pro) - X(Lys) - X(Gln) - X(Ala) - X(Thr) - X(Glu) -
                              70
X(Leu) - X(Lys) - X(Asp) - X(Leu) - X(Gln) -   Cys  -

X(Leu) - X(Glu) - X(Asp) - X(Glu) - X(Leu) - X(Gly) -
            80
X(Pro) - X(Leu) - X(Arg) - X(His) - X(Val) - X(Leu) -
                                                90
X(Asp) - X(Leu) - X(Thr) - X(Gln) - X(Ser) - X(Lys) -

X(Ser) - X(Phe) - X(Gln) - X(Leu) - X(Glu) - X(Asp) -
                              100
X(Ala) - X(Glu) - X(Asn) - X(Phe) - X(Ile) - X(Ser) -
```

```
X(Asn) - X(Ile) - X(Arg) - X(Val) - X(Thr) - X(Val) -
          110
X(Val) - X(Lys) - X(Leu) - X(Lys) - X(Gly) - X(Ser) -
                                      120
X(Asp) - X(Asn) - X(Thr) - X(Phe) - X(Glu) -  Cys   -

X(Gln) - X(Phe) - X(Asp) - X(Asp) - X(Glu) - X(Ser) -
                            130
X(Ala) - X(Thr) - X(Val) - X(Val) - X(Asp) - X(Phe) -

X(Leu) - X(Arg) - X(Arg) - X(Trp) - X(Ile) - X(Ala) -
          140
X(Phe) - X(Cys) - X(Gln) - X(Ser) - X(Ile) - X(Ile) -

X(Ser) - X(Thr) - X(Ser) - X(Pro) - X(Gln)
```

wherein $Cys^{72}$ and $Cys^{120}$ are neither substituted nor deleted, at least one deletion, substitution and/or insertion has been made, and

X(Ser) represents Ser, Thr, Gly, or Asn;
X(Arg) represents Arg, His, Gln, Lys, or Glu;
X(Leu) represents Leu, Ile, Phe, Tyr, Met, or Val;
X(Pro) represents Pro, Gly, Ala, or Thr;
X(Thr) represents Thr, Pro, Ser, Ala, Gly, His, or Gln;
X(Ala) represents Ala, Gly, Thr, or Pro;
X(Val) represents Val, Met, Tyr, Phe, Ile, or Leu;
X(Gly) represents Gly, Ala, Thr, Pro, or Ser;
X(Ile) represents Ile, Met, Tyr, Phe, Val, or Leu;
X(Phe) represents Phe, Trp, Met, Tyr, Ile, Val, or Leu;
X(Tyr) represents Tyr, Trp, Met, Phe, Ile, Val, or Leu;
X(His) represents His, Glu, Lys, Gln, Thr, or Arg;
X(Gln) represents Gln, Glu, Lys, Asn, His, Thr, or Arg;
X(Asn) represents Asn, Glu, Asp, Gln, or Ser;
X(Lys) represents Lys, Glu, Gln, His, or Arg;
X(Asp) represents Asp, Glu, or Asn;
X(Glu) represents Glu, Asp, Lys, Asn, Gln, His, or Arg; and
X(Met) represents Met, Phe, Ile, Val, Leu, or Tyr.
   2. The murine interleukin-2 muteins of claim 1 wherein:
X(Ser) is Ser;
X(Arg) represents Arg, His, or Lys;
X(Leu) represents Leu, Ile, Phe, or Met;
X(Pro) represents Pro or Ala;
X(Thr) is Thr;
X(Ala) represents Ala or Pro;
X(Val) represents Val, Met, or Ile;
X(Gly) is Gly;
X(Ile) represents Ile, Met, Phe, Val, or Leu;
X(Phe) represents Phe, Met, Tyr, Ile, or Leu;
X(Thr) represents Tyr or Phe;
X(His) represents His, Gln, or Arg;
X(Gln) represents Gln, Glu, or His;
X(Asn) represents Asn or Asp;
X(Lys) represents Lys or Arg;
X(Asp) represents Asp or Asn;
X(Glu) represents Glu or Gln; and
X(Met) represents Met, Phe, Ile, Val, or Leu.
   3. The murine interleukin-2 muteins of claim 1 wherein:
X(Arg) is Arg;
X(Leu) represents Leu, Ile, or Met;
X(Pro) is Pro;
X(Ala) is Ala;
X(Val) is Val;
X(Ile) represents Ile, Met, or Leu;

X(Phe) is Phe;
X(Tyr) is Tyr;
X(His) is His;
X(Gln) is Gln;
X(Asn) is Asn;
X(Lys) is Lys;
X(Asp) is Asp;
X(Glu) is Glu; and
X(Met) represents Met, Ile, or Leu.

4. Murine interleukin-2 muteins comprising polypeptide sequences selected from 12-fold deleted (14-25), 1-fold deleted (26-140), 5-fold deleted (141-149), 5-fold substituted (1-25), 3-fold substituted (26-38), 3-fold substituted (39-140), 5-fold substituted (141-149), 5-fold inserted (0-13), 1-fold inserted (14-149) polypeptides of the formula:

$$X(Ala) - X(Pro) - X(Thr) - X(Ser) - X(Ser) - X(Ser) -$$
$$10$$
$$X(Thr) - X(Ser) - X(Ser) - X(Ser) - X(Thr) - X(Ala) -$$

$$X(Glu) - X(Ala) - X(Gln) - X(Gln) - X(Gln) - X(Gln) -$$
$$20$$
$$X(Gln) - X(Gln) - X(Gln) - X(Gln) - X(Gln) - X(Gln) -$$
$$30$$
$$X(Gln) - X(Gln) - X(His) - X(Leu) - X(Glu) - X(Gln) -$$

$$X(Leu) - X(Leu) - X(Met) - X(Asp) - X(Leu) - X(Gln) -$$
$$40$$
$$X(Glu) - X(Leu) - X(Leu) - X(Ser) - X(Arg) - X(Met) -$$

$$X(Glu) - X(Asn) - X(Tyr) - X(Arg) - X(Asn) - X(Leu) -$$
$$50$$
$$X(Lys) - X(Leu) - X(Pro) - X(Arg) - X(Met) - X(Leu) -$$
$$60$$
$$X(Thr) - X(Phe) - X(Lys) - X(Phe) - X(Tyr) - X(Leu) -$$

$$X(Pro) - X(Lys) - X(Gln) - X(Ala) - X(Thr) - X(Glu) -$$

14

X(Leu) - X(Lys) - X(Asp) - X(Leu) - X(Gln) -   Cys  -

X(Leu) - X(Glu) - X(Asp) - X(Glu) - X(Leu) - X(Gly) -

X(Pro) - X(Leu) - X(Arg) - X(His) - X(Val) - X(Leu) -

X(Asp) - X(Leu) - X(Thr) - X(Gln) - X(Ser) - X(Lys) -

X(Ser) - X(Phe) - X(Gln) - X(Leu) - X(Glu) - X(Asp) -

X(Ala) - X(Glu) - X(Asn) - X(Phe) - X(Ile) - X(Ser) -

X(Asn) - X(Ile) - X(Arg) - X(Val) - X(Thr) - X(Val) -

X(Val) - X(Lys) - X(Leu) - X(Lys) - X(Gly) - X(Ser) -

X(Asp) - X(Asn) - X(Thr) - X(Phe) - X(Glu) -   Cys  -

X(Gln) - X(Phe) - X(Asp) - X(Asp) - X(Glu) - X(Ser) -

X(Ala) - X(Thr) - X(Val) - X(Val) - X(Asp) - X(Phe) -

X(Leu) - X(Arg) - X(Arg) - X(Trp) - X(Ile) - X(Ala) -

X(Phe) - X(Cys) - X(Gln) - X(Ser) - X(Ile) - X(Ile) -

X(Ser) - X(Thr) - X(Ser) - X(Pro) - X(Gln)

wherein $Cys^{72}$ and $Cys^{120}$ are neither substituted nor deleted, at least one deletion, substitution and/or insertion has been made, and

X(Ser) represents Ser, Thr, Gly, or Asn;

X(Arg) represents Arg, His, Gln, Lys, or Glu;

X(Leu) represents Leu, Ile, Phe, Tyr, Met, or Val;

X(Pro) represents Pro, Gly, Ala, or Thr;

X(Thr) represents Thr, Pro, Ser, Ala, Gly, His, or Gln;

X(Ala) represents Ala, Gly, Thr, or Pro;

X(Val) represents Val, Met, Tyr, Phe, Ile, or Leu;

X(Gly) represents Gly, Ala, Thr, Pro, or Ser;

X(Ile) represents Ile, Met, Tyr, Phe, Val, or Leu;

X(Phe) represents Phe, Trp, Met, Tyr, Ile, Val, or Leu;

X(Tyr) represents Tyr, Trp, Met, Phe, Ile, Val, or Leu;

X(His) represents His, Glu, Lys, Gln, Thr, or Arg;

X(Gln) represents Gln, Glu, Lys, Asn, His, Thr, or Arg;

X(Asn) represents Asn, Glu, Asp, Gln, or Ser;

X(Lys) represents Lys, Glu, Gln, His, or Arg;

X(Asp) represents Asp, Glu, or Asn;

X(Glu) represents Glu, Asp, Lys, Asn, Gln, His, or Arg; and

X(Met) represents Met, Phe, Ile, Val, Leu, or Tyr.

5. The murine interleukin-2 muteins of claim 4 wherein:

X(Ser) is Ser;

X(Arg) represents Arg, His, or Lys;

X(Leu) represents Leu, Ile, Phe, or Met;

X(Pro) represents Pro or Ala;

X(Thr) is Thr;

X(Ala) represents Ala or Pro;

X(Val) represents Val, Met, or Ile;

X(Gly) is Gly;

X(Ile) represents Ile, Met, Phe, Val, or Leu;

X(Phe) represents Phe, Met, Tyr, Ile, or Leu;

X(Thr) represents Tyr or Phe;

X(His) represents His, Gln, or Arg;

X(Gln) represents Gln, Glu, or His;

X(Asn) represents Asn or Asp;

X(Lys) represents Lys or Arg;

X(Asp) represents Asp or Asn;

X(Glu) represents Glu or Gln; and

X(Met) represents Met, Phe, Ile, Val, or Leu.

6. The murine interleukin-2 muteins of claim 5 wherein:

X(Arg) is Arg;

X(Leu) represents Leu, Ile, or Met;

X(Pro) is Pro;

X(Ala) is Ala;

X(Val) is Val;

X(Ile) represents Ile, Met, or Leu;

X(Phe) is Phe;

X(Tyr) is Tyr;

X(His) is His;

X(Gln) is Gln;

X(Asn) is Asn;

X(Lys) is Lys;

X(Asp) is Asp;

X(Glu) is Glu; and

X(Met) represents Met, Ile, or Leu.

7. Murine interleukin-2 muteins comprising popypeptide sequences selected from the group consisting of 12-fold deleted (14-25), 5-fold deleted (141-149), 3-fold substituted (1-25), 2-fold substituted (26-38), 3-fold substituted (141-149) polypeptides of the formula:

$$X(Ala) - X(Pro) - X(Thr) - X(Ser) - X(Ser) - X(Ser) -$$
$$10$$
$$X(Thr) - X(Ser) - X(Ser) - X(Ser) - X(Thr) - X(Ala) -$$
$$X(Glu) - X(Ala) - X(Gln) - X(Gln) - X(Gln) - X(Gln) -$$
$$20$$
$$X(Gln) - X(Gln) - X(Gln) - X(Gln) - X(Gln) - X(Gln) -$$
$$30$$
$$X(Gln) - X(Gln) - X(His) - X(Leu) - X(Glu) - X(Gln) -$$

```
X(Leu) - X(Leu) - X(Met) - X(Asp) - X(Leu) - X(Gln) -
                        40
X(Glu) - X(Leu) - X(Leu) - X(Ser) - X(Arg) - X(Met) -

X(Glu) - X(Asn) - X(Tyr) - X(Arg) - X(Asn) - X(Leu) -
          50
X(Lys) - X(Leu) - X(Pro) - X(Arg) - X(Met) - X(Leu) -
                                              60
X(Thr) - X(Phe) - X(Lys) - X(Phe) - X(Tyr) - X(Leu) -

X(Pro) - X(Lys) - X(Gln) - X(Ala) - X(Thr) - X(Glu) -
                    70
X(Leu) - X(Lys) - X(Asp) - X(Leu) - X(Gln) -   Cys   -

X(Leu) - X(Glu) - X(Asp) - X(Glu) - X(Leu) - X(Gly) -
          80
X(Pro) - X(Leu) - X(Arg) - X(His) - X(Val) - X(Leu) -
                                              90
X(Asp) - X(Leu) - X(Thr) - X(Gln) - X(Ser) - X(Lys) -

X(Ser) - X(Phe) - X(Gln) - X(Leu) - X(Glu) - X(Asp) -
                            100
X(Ala) - X(Glu) - X(Asn) - X(Phe) - X(Ile) - X(Ser) -

X(Asn) - X(Ile) - X(Arg) - X(Val) - X(Thr) - X(Val) -
          110
X(Val) - X(Lys) - X(Leu) - X(Lys) - X(Gly) - X(Ser) -
                                              120
X(Asp) - X(Asn) - X(Thr) - X(Phe) - X(Glu) -   Cys   -

X(Gln) - X(Phe) - X(Asp) - X(Asp) - X(Glu) - X(Ser) -
                            130
X(Ala) - X(Thr) - X(Val) - X(Val) - X(Asp) - X(Phe) -

X(Leu) - X(Arg) - X(Arg) - X(Trp) - X(Ile) - X(Ala) -
          140
X(Phe) - X(Cys) - X(Gln) - X(Ser) - X(Ile) - X(Ile) -

X(Ser) - X(Thr) - X(Ser) - X(Pro) - X(Gln)
```

wherein $Cys^{72}$ and $Cys^{120}$ are neither substituted nor deleted, at least one deletion and/or substitution has been made, and

X(Ser) represents Ser, Thr, Gly, or Asn;
X(Arg) represents Arg, His, Gln, Lys, or Glu;
X(Leu) represents Leu, Ile, Phe, Tyr, Met, or Val;
X(Pro) represents Pro, Gly, Ala, or Thr;
X(Thr) represents Thr, Pro, Ser, Ala, Gly, His, or Gln;
X(Ala) represents Ala, Gly, Thr, or Pro;
X(Val) represents Val, Met, Tyr, Phe, Ile, or Leu;
X(Gly) represents Gly, Ala, Thr, Pro, or Ser;
X(Ile) represents Ile, Met, Tyr, Phe, Val, or Leu;
X(Phe) represents Phe, Trp, Met, Tyr, Ile, Val, or Leu;
X(Tyr) represents Tyr, Trp, Met, Phe, Ile, Val, or Leu;
X(His) represents His, Glu, Lys, Gln, Thr, or Arg;
X(Gln) represents Gln, Glu, Lys, Asn, His, Thr, or Arg;
X(Asn) represents Asn, Glu, Asp, Gln, or Ser;
X(Lys) represents Lys, Glu, Gln, His, or Arg;
X(Asp) represents Asp, Glu, or Asn;

X(Glu) represents Glu, Asp, Lys, Asn, Gln, His, or Arg; and
X(Met) represents Met, Phe, Ile, Val, Leu, or Tyr.

8. The murine interleukin-2 muteins of claim 7 wherein:
X(Ser) is Ser;
X(Arg) represents Arg, His, or Lys;
X(Leu) represents Leu, Ile, Phe, or Met;
X(Pro) represents Pro or Ala;
X(Thr) is Thr;
X(Ala) represents Ala or Pro;
X(Val) represents Val, Met, or Ile;
X(Gly) is Gly;
X(Ile) represents Ile, Met, Phe, Val, or Leu;
X(Phe) represents Phe, Met, Tyr, Ile, or Leu;
X(Thr) represents Tyr or Phe;
X(His) represents His, Gln, or Arg;
X(Gln) represents Gln, Glu, or His;
X(Asn) represents Asn or Asp;
X(Lys) represents Lys or Arg;
X(Asp) represents Asp or Asn;
X(Glu) represents Glu or Gln; and
X(Met) represents Met, Phe, Ile, Val, or Leu.

9. The murine interleukin-2 muteins of claim 8 wherein:
X(Arg) is Arg;
X(Leu) represents Leu, Ile, or Met;
X(Pro) is Pro;
X(Ala) is Ala;
X(Val) is Val;
X(Ile) represents Ile, Met, or Leu;
X(Phe) is Phe;
X(Tyr) is Tyr;
X(His) is His;
X(Gln) is Gln;
X(Asn) is Asn;
X(Lys) is Lys;
X(Asp) is Asp;
X(Glu) is Glu; and
X(Met) represents Met, Ile, or Leu.

GAATTCTCATGTTTACAGCTTATCTCGGAGCTGCATGTGTCAGAGCTTTCACCGTCATCACCGAAA
EcoRI

CGCGCAGGCAAGCTGTTGACAATTAATCATCGGCTCGTATAATGTGTGGAATTGTGAGCGGATAAC

AATTTCACACAGGAAACAGGATCG**TAAGGAGGT**TTAAC ATG AGC TCG CCC GGG GAT CCG
                                                SstI        SmaI BamHI

TCG ACC TGC AGG CGG AGA ACT GGT AGG TAT GGA AGA TCT CTA GAA GCT TT
SalI     PstI                                BglII  XbaI HindIII

## FIGURE 1A

GAATTCGAGCTCGCCCAGGTTTAACG**TAAGGAGGT**TTAACCATCG ATG GAT CCG TCG ACC
EcoRI SstI              –              ClaI    BamHI    SalI

TGC AGG CGG AGA ACT GGT AGG TAT GGA AGA TCT CTA GAA GCT TT
  PstI                            BglII  XbaI HindIII

## FIGURE 1B

GAATTCCCGGGGATCGCTAGGGTGCCGAGCGCATCTCGACTGCACGGTGCACCAATGCTTCTGGCG

EcoRI SmaI

TCAGGTAGTTATTGGAAAGCTGTGGTATGGCTGTGCAGGTCGTAAATCACTGCATAACTCGCTGCT

GCTCAAGGCGCACTCCCGTTCTGGATAATGTTTTTTGCGCCGACATCATAACGGTTCTGGCAAATA

TTCTGAAATGAGCTGTTGACAATTAATCATCGAACTAGTTAACTAGTACGCAAGTTCACGTAAA**AA**

**GG**GTATCGACA        ATG AAA GCA ATT TTC GTA CTG AAA GGT TCA CTG GAC

AGA GAT CCA C**AG** **GAG** ACT TTC TG ATG AGC TCG CCC GGG GAT CCG TCG ACC
                                   SstI          SmaI BamHI  SalI

TGC AGG CGG AGA ACT GGT AGG TAT GGA AGA TCT CTA GAA GCT TT
                                    BglII   XbaI HindIII

FIGURE 1C

```
                                      Xba       Pst       Nru           Sph
                                    10        20        30        40        50        60
● mIL2          APTSSSTSSSTAEAQQQQQQQQQQQQQQHLEQLLMDLQELLSRMENYRNLKLPRMLTFKFYLP
● mIL2-7      mnsAPTSSSTSSSTAEAQQQQQQQQQQQQQQHLEQLLMDLQELLSRMENYRNLKLPRMLTFKFYLP
  ΔSst6 -      mAPTSSSTSSSTAEAQQQQQQQQQQQQQQHLEQLLMDLh    LSRMENYRNLKLPRMLTFKFYLP
  ΔSst1 -      mAPTSSSTSSSTAEAQQQQQQQQQQQQQQHLEQLLMDL     LSRMENYRNLKLPRMLTFKFYLP
● ΔSst5 +      mAPTSSSTSSSTAEAQQQQQQQQQQQQQQHLEQLLMD      LSRMENYRNLKLPRMLTFKFYLP
  ΔSst3 -      mAPTSSSTSSSTAEAQQQQQQQQQQQQQQHLEQLLMDLQg   RMENYRNLKLPRMLTFKFYLP
● ΔSph1        mAPTSSSTSSSTAEAQQQQQQQQQQQQQQHLEQLLMDLQELLSRMENYRNLKLPR   TFKFYLP
  ΔSph8        mAPTSSSTSSSTAEAQQQQQQQQQQQQQQHLEQLLMDLQELLSRMENYRNLKLP
● ΔSph10       mAPTSSSTSSSTAEAQQQQQQQQQQQQQQHLEQLLMDLQELLSRMENYRNLm    TFKFYLP
● Δ43,97            mAEAQQQQQQQQQQQQQQHLEQLLMDLQELLSRMENYRNLKLPRMLTFKFYLP
● Δ44,70              mAQQQQQQQQQQQQQQHLEQLLMDLQELLSRMENYRNLKLPRMLTFKFYLP
● ΔHis                    mHLEQLLMDLQELLSRMENYRNLKLPRMLTFKFYLP
  ΔHis-ΔQ                 mHLEQLLMDLQELLSRMENYRNLKLPRMLTFKFYLP
● ΔPvuII                     mLLMDLQELLSRMENYRNLKLPRMLTFKFYLP
  ΔMet                         MDLQELLSRMENYRNLKLPRMLTFKFYLP
  ΔMet-S                       MDLQELLSRMENYRNLKLPRMLTskFYLP
  Δ36                               mENYRNLKLPRMLTFKFYLP
  Δ16                                 mRNLKLPRMLTFKFYLP
● ΔGLN1-2      mAPTSSSTSSSTAqA            HLEQLLMDLQELLSRMENYRNLKLPRMLTFKFYLP
● ΔGLN1b       mAPTSSSTSSSTAEA            qvEQLLMDLQELLSRMENYRNLKLPRMLTFKFYLP
● ΔGLN2-2      mAPTSSSTSSSTAEA            HLqQLLiDLhELLSRMENYRNLKLPRMLTFKFYLP
● ΔGLN3a       mArTSSSsSSSTAEA            HLEQLLMDLeELLSRMENYRNLKLPRMLTFKFYLP
● ΔGLN4a       mAPTStSTSSSTAEA           HLEhLLiDLQELLSRMENYRNLKLPRMLTFKFYLP
● ΔGLN6-2      mpPTSrSTSSSTAEA           HLEQLLMDLQELLSRMENYRNLKLPRMLTFKFYLP
● ΔGLN7-2      mAPTSSSTStSTpEp           HLqhLLMDLQELLSRMENYRNLKLPRMLTFKFYLP
● ΔGLN3-2      mAPTSSSTSSSTAEA           HLEQLfMDLQELLSRMENYRNLKLPRMLTFKFYLP
● ΔGLN6        mAPTSSSTStSTAEA           HLdhLLMDLQqLLSRMENYRNLKLPRMLTFKFYLP
● ΔGLN2a       mAPTSSSTSSSTpEA           qvEQLLMDvQELLSRMENYRNLKLPRMLTFKFYLP
● ΔGLN4-2      mAPTSSSTSSSTAEp           HLEhLLMhLQELLSRMENYRNLKLPRMLTFKFYLP
● ΔGLN9b       mArTSrSTSScTgEg           HvEQLLMDLeELLSRMENYRNLKLPRMLTFKFYLP
  ΔGLN15-2     mAPTSSSTSSSTAqA           HLqQLfMhLQELLSRMENYRNLKLPRMLTFKFYLP
● 7-1   CT     mAPTSSSTSSSTAEAQQQQQQQQQQQQQQHLEQLLMDLQELLSRMENYRNLKLPRMLTFKFYLP
● 10-1  CT     mAPTSSSTSSSTAEAQQQQQQQQQQQQQQHLEQLLMDLQELLSRMENYRNLKLPRMLTFKFYLP
  ΔHIII 17     mAPTSSSTSSSTAEAQQQQQQQQQQQQQQHLEQLLMDLQELLSRMENYRNLKLPRMLTFKFYLP
  ΔHIII 19     mAPTSSSTSSSTAEAQQQQQQQQQQQQQQHLEQLLMDLQELLSRMENYRNLKLPRMLTFKFYLP
  ΔHIII        mAPTSSSTSSSTAEAQQQQQQQQQQQQQQHLEQLLMDLQELLSRMENYRNLKLPRMLTFKFYLP
  ΔXho 5       mAPTSSSTSSSTAEAQQQQQQQQQQQQQQHLEQLLMDLQELLSRMENYRNLKLPRMLTFKFYLP
  ΔXho 7       mAPTSSSTSSSTAEAQQQQQQQQQQQQQQHLEQLLMDLQELLSRMENYRNLKLPRMLTFKFYLP
  ΔXho 8       mAPTSSSTSSSTAEAGQQQQQQQQQQQQQHLEQLLMDLQELLSRMENYRNLKLPRMLTFKFYLP
  Mlu12-1      mAPTSSSTSSSTAEAQQGQQQQQQQQQQQHLEQLLMDLQELLSRMENYRNLKLPRMLTFKFYLP
  ΔMlu 22      mAPTSSSTSSSTAEAQQQQQQQQQQQQQQHLEQLLMDLQELLSRMENYRNLKLPRMLTFKFYLP
  Bam-2        mAPTSSSTSSSTAEAQQQQQQQQQQQQQQHLEQLLMDLQELLSRMENYRNLKLPRMLTFKFYLP
  Bam-5        mAPTSSSTSSSTAEAQQQQQQQQQQQQQQHLEQLLMDLQELLSRMENYRNLKLPRMLTFKFYLP
  ΔBam F1      mAPTSSSTSSSTAEAQQQQQQQQQQQQQQHLEQLLMDLQELLSRMENYRNLKLPRMLTFKFYLP
  ΔBam 12      mAPTSSSTSSSTAEAQQQQQQQQQQQQQQHLEQLLMDLQELLSRMENYRNLKLPRMLTFKFYLP
  2-1   CT     mAPTSSSTSSSTAEAQQQQQQQQQQQQQQHLEQLLMDLQELLSRMENYRNLKLPRMLTFKFYLP
  14-2 CT      mAPTSSSTSSSTAEAQQQQQQQQQQQQQQHLEQLLMDLQELLSRMENYRNLKLPRMLTFKFYLP
  ΔSal D2      mAPTSSSTSSSTAEAQQQQQQQQQQQQQQHLEQLLMDLQELLSRMENYRNLKLPRMLTFKFYLP
  ΔSnaBI 3     mAPTSSSTSSSTAEAQQQQQQQQQQQQQQHLEQLLMDLQELLSRMENYRNLKLPRMLTFKFYLP
  ΔSnaBI 2     mAPTSSSTSSSTAEAQQQQQQQQQQQQQQHLEQLLMDLQELLSRMENYRNLKLPRMLTFKFYLP
● ΔScaI 7      mAPTSSSTSSSTAEAQQQQQQQQQQQQQQHLEQLLMDLQELLSRMENYRNLKLPRMLTFKFYLP
● ΔScaI 12     mAPTSSSTSSSTAEAQQQQQQQQQQQQQQHLEQLLMDLQELLSRMENYRNLKLPRMLTFKFYLP
● ΔScaI 5      mAPTSSSTSSSTAEAQQQQQQQQQQQQQQHLEQLLMDLQELLSRMENYRNLKLPRMLTFKFYLP
● mIL2          APTSSSTSSSTAEAQQQQQQQQQQQQQQHLEQLLMDLQELLSRMENYRNLKLPRMLTFKFYLP
                                    10        20        30        40        50        60
```

## FIGURE 2

(A)

```
                 Sph           Bgl                        III Xho      0267795Bam
                 50        60        70        80        90        100       110       120
●mIL2            LPRMLTFKFYLPKQATELKDLQCLEDELGPLRHVLDLTQSKSFQLEDAENFISNIRVTVVKLKGSDNTFECQF
●mIL2-7          LPRMLTFKFYLPKQATELKDLQCLEDELGPLRHVLDLTQSKSFQLEDAENFISNIRVTVVKLKGSDNTFECQF
 ΔSst6 -         LPRMLTFKFYLPKQATELKDLQCLEDELGPLRHVLDLTQSKSFQLEDAENFISNIRVTVVKLKGSDNTFECQF
 ΔSst1 -         LPRMLTFKFYLPKQATELKDLQCLEDELGPLRHVLDLTQSKSFQLEDAENFISNIRVTVVKLKGSDNTFECQF
●ΔSst5 +         LPRMLTFKFYLPKQATELKDLQCLEDELGPLRHVLDLTQSKSFQLEDAENFISNIRVTVVKLKGSDNTFECQF
 ΔSst3 -         LPRMLTFKFYLPKQATELKDLQCLEDELGPLRHVLDLTQSKSFQLEDAENFISNIRVTVVKLKGSDNTFECQF
●ΔSph1           LPR   TFKFYLPKQATELKDLQCLEDELGPLRHVLDLTQSKSFQLEDAENFISNIRVTVVKLKGSDNTFECQF
 ΔSph8           LP              ATELKDLQCLEDELGPLRHVLDLTQSKSFQLEDAENFISNIRVTVVKLKGSDNTFECQF
●ΔSph10              TFKFYLPKQATELKDLQCLEDELGPLRHVLDLTQSKSFQLEDAENFISNIRVTVVKLKGSDNTFECQF
●Δ43,97          LPRMLTFKFYLPKQATELKDLQCLEDELGPLRHVLDLTQSKSFQLEDAENFISNIRVTVVKLKGSDNTFECQF
●Δ44,70          LPRMLTFKFYLPKQATELKDLQCLEDELGPLRHVLDLTQSKSFQLEDAENFISNIRVTVVKLKGSDNTFECQF
●ΔHis            LPRMLTFKFYLPKQATELKDLQCLEDELGPLRHVLDLTQSKSFQLEDAENFISNIRVTVVKLKGSDNTFECQF
 ΔHis-ΔQ         LPRMLTFKFYLPK ATELKDLQCLEDELGPLRHVLDLTQSKSFQLEDAENFISNIRVTVVKLKGSDNTFECQF
●ΔPvuII          LPRMLTFKFYLPKQATELKDLQCLEDELGPLRHVLDLTQSKSFQLEDAENFISNIRVTVVKLKGSDNTFECQF
 ΔMet            LPRMLTFKFYLPKQATELKDLQCLEDELGPLRHVLDLTQSKSFQLEDAENFISNIRVTVVKLKGSDNTFECQF
 ΔMet-S          LPRMLTsKFYLPKQATELKDLQCLEDELGPLRHVLDLTQSKSFQLEDAENFISNIRVTVVKLKGSDNTFECQF
 Δ36             LPRMLTFKFYLPKQATELKDLQCLEDELGPLRHVLDLTQSKSFQLEDAENFISNIRVTVVKLKGSDNTFECQF
 Δ16             LPRMLTFKFYLPKQATELKDLQCLEDELGPLRHVLDLTQSKSFQLEDAENFISNIRVTVVKLKGSDNTFECQF
●ΔGLN1-2         LPRMLTFKFYLPKQATELKDLQCLEDELGPLRHVLDLTQSKSFQLEDAENFISNIRVTVVKLKGSDNTFECQF
●ΔGLN1b          LPRMLTFKFYLPKQATELKDLQCLEDELGPLRHVLDLTQSKSFQLEDAENFISNIRVTVVKLKGSDNTFECQF
●ΔGLN2-2         LPRMLTFKFYLPKQATELKDLQCLEDELGPLRHVLDLTQSKSFQLEDAENFISNIRVTVVKLKGSDNTFECQF
●ΔGLN3a          LPRMLTFKFYLPKQATELKDLQCLEDELGPLRHVLDLTQSKSFQLEDAENFISNIRVTVVKLKGSDNTFECQF
●ΔGLN4a          LPRMLTFKFYLPKQATELKDLQCLEDELGPLRHVLDLTQSKSFQLEDAENFISNIRVTVVKLKGSDNTFECQF
●ΔGLN6-2         LPRMLTFKFYLPKQATELKDLQCLEDELGPLRHVLDLTQSKSFQLEDAENFISNIRVTVVKLKGSDNTFECQF
●ΔGLN7-2         LPRMLTFKFYLPKQATELKDLQCLEDELGPLRHVLDLTQSKSFQLEDAENFISNIRVTVVKLKGSDNTFECQF
●ΔGLN3-2         LPRMLTFKFYLPKQATELKDLQCLEDELGPLRHVLDLTQSKSFQLEDAENFISNIRVTVVKLKGSDNTFECQF
●ΔGLN6           LPRMLTFKFYLPKQATELKDLQCLEDELGPLRHVLDLTQSKSFQLEDAENFISNIRVTVVKLKGSDNTFECQF
●ΔGLN2a          LPRMLTFKFYLPKQATELKDLQCLEDELGPLRHVLDLTQSKSFQLEDAENFISNIRVTVVKLKGSDNTFECQF
●ΔGLN4-2         LPRMLTFKFYLPKQATELKDLQCLEDELGPLRHVLDLTQSKSFQLEDAENFISNIRVTVVKLKGSDNTFECQF
●ΔGLN9b          LPRMLTFKFYLPKQATELKDLQCLEDELGPLRHVLDLTQSKSFQLEDAENFISNIRVTVVKLKGSDNTFECQF
 ΔGLN15-2        LPRMLTFKFYLPKQATELKDLQCLEDELGPLRHVLDLTQSKSFQLEDAENFISNIRVTVVKLKGSDNTFECQF
●7-1  CT         LPRMLTFKFYLPKQATELKDLQCLEDELGPLRHVLDLTQSKSFQLEDAEkFISNIRVTVVKLKGSDNTFECQF
●10-1 CT         LPRMLTFKFYLPKQATELKDLQCLEDELGPLRHVLDLTQSKSyQLEDAENFISNIRVTVVKLKGSDNTFECQF
 ΔHIII 17        LPRMLTFKFYLPKQATELKDLQCLEDELGPLRHVLDLTQS.
 ΔHIII 19        LPRMLTFKFYLPKQATELKDLQCLEDELGPLRHVLDLTQSeSIaLavvIqrrdwenpgVtqLnrIaahppfas
 ΔHIII           LPRMLTFKFYLPKQATELKDLQCLEDELGPLRHVLDLTQSKSIaLavvIqrrdwenpgVtqLnrIaahppfas
 ΔXho 5          LPRMLTFKFYLPKQATELKDLQCLEDELGPLRHVLDLTQSKS.
 ΔXho 7          LPRMLTFKFYLPKQATELKDLQCLEDELGPLRHVLDLTQSKSFe.
 ΔXho 8          LPRMLTFKFYLPKQATELKDLQCLEDELGPLRHVLDLTQSKSFQaEIgtgrrftts.
 Mlu12-1         LPRMLTFKFYLPKQATELKDLQCLEDELGPLRHVLDLTQSKSFQLEDAEN.
 ΔMlu 22         LPRMLTFKFYLPKQATELKDLQCLEDELGPLRHVLDLTQSKSFQLEDAENFISe.
 Bam-2           LPRMLTFKFYLPKQATELKDLQCLEDELGPLRHVLDLTQSKSFQLEDAENFISNvssTtnILIIStsyvdgsl
 Bam-5           LPRMLTFKFYLPKQATELKDLQCLEDELGPLRHVLDLTQSKSFQLEDAENFISNIRVsVrrricyccrlpt.
 ΔBam F1         LPRMLTFKFYLPKQATELKDLQCLEDELGPLRHVLDLTQSKSFQLEDAENFISNIRVTgelgtGrrfTts.
 ΔBam 12         LPRMLTFKFYLPKQATELKDLQCLEDELGPLRHVLDLTQSKSFQLEDAENFISNIRVTVmrawawpsI.
 2-1  CT         LPRMLTFKFYLPKQATELKDLQCLEDELGPLRHVLDLTQSKSyQLEDAENFISNIRVTgVKr.
 14-2 CT         LPRMLTFKFYLPKQATELKDLQCLEDELGPLRHVLDLTQSKSFQLEDsENFISNIRVTVVKLKGSgqhFrmsv
 ΔSal D2         LPRMLTFKFYLPKQATELKDLQCLEDELGPLRHVLDLTQSKSFQLEDAENFISNIRVTVVKLKGSDNTFECQF
 ΔSnaBI 3        LPRMLTFKFYLPKQATELKDLQCLEDELGPLRHVLDLTQSKSFQLEDAENFISNIRVTVVKLKGSDNTFECQF
 ΔSnaBI 2        LPRMLTFKFYLPKQATELKDLQCLEDELGPLRHVLDLTQSKSFQLEDAENFISNIRVTVVKLKGSDNTFECQF
●ΔScaI 7         LPRMLTFKFYLPKQATELKDLQCLEDELGPLRHVLDLTQSKSFQLEDAENFISNIRVTVVKLKGSDNTFECQF
●ΔScaI 12        LPRMLTFKFYLPKQATELKDLQCLEDELGPLRHVLDLTQSKSFQLEDAENFISNIRVTVVKLKGSDNTFECQF
●ΔScaI 5         LPRMLTFKFYLPKQATELKDLQCLEDELGPLRHVLDLTQSKSFQLEDAENFISNIRVTVVKLKGSDNTFECQF
●mIL2            LPRMLTFKFYLPKQATELKDLQCLEDELGPLRHVLDLTQSKSFQLEDAENFISNIRVTVVKLKGSDNTFECQF
                 50        60        70        80        90        100       110       120
```

FIGURE 2

(B)

|  |  | Bam |  | Sal Sna |  | Sca |
|---|---|---|---|---|---|---|

```
                    Bam                Sal Sna        Sca
                  110      120      130      140
● mIL2            KLKGSDNTFECQFDDESATVVDFLRRWIAFCQSIISTSPQ.
● mIL2-7          KLKGSDNTFECQFDDESATVVDFLRRWIAFCQSIISTSPQ.
  ΔSst6 -         KLKGSDNTFECQFDDESATVVDFLRRWIAFCQSIISTSPQ.
  ΔSst1 -         KLKGSDNTFECQFDDESATVVDFLRRWIAFCQSIISTSPQ.
● ΔSst5 +         KLKGSDNTFECQFDDESATVVDFLRRWIAFCQSIISTSPQ.
  ΔSst3 -         KLKGSDNTFECQFDDESATVVDFLRRWIAFCQSIISTSPQ.
● ΔSph1           KLKGSDNTFECQFDDESATVVDFLRRWIAFCQSIISTSPQ.
  ΔSph8           KLKGSDNTFECQFDDESATVVDFLRRWIAFCQSIISTSPQ.
● ΔSph10          KLKGSDNTFECQFDDESATVVDFLRRWIAFCQSIISTSPQ.
● Δ43,97          KLKGSDNTFECQFDDESATVVDFLRRWIAFCQSIISTSPQ.
● Δ44,70          KLKGSDNTFECQFDDESATVVDFLRRWIAFCQSIISTSPQ.
● ΔHis            KLKGSDNTFECQFDDESATVVDFLRRWIAFCQSIISTSPQ.
  ΔHis-ΔQ         KLKGSDNTFECQFDDESATVVDFLRRWIAFCQSIISTSPQ.
● ΔPvuII          KLKGSDNTFECQFDDESATVVDFLRRWIAFCQSIISTSPQ.
  ΔMet            KLKGSDNTFECQFDDESATVVDFLRRWIAFCQSIISTSPQ.
  ΔMet-S          KLKGSDNTFECQFDDESATVVDFLRRWIAFCQSIISTSPQ.
  Δ36             KLKGSDNTFECQFDDESATVVDFLRRWIAFCQSIISTSPQ.
  Δ16             KLKGSDNTFECQFDDESATVVDFLRRWIAFCQSIISTSPQ.
● ΔGLN1-2         KLKGSDNTFECQFDDESATVVDFLRRWIAFCQSIISTSPQ.
● ΔGLN1b          KLKGSDNTFECQFDDESATVVDFLRRWIAFCQSIISTSPQ.
● ΔGLN2-2         KLKGSDNTFECQFDDESATVVDFLRRWIAFCQSIISTSPQ.
● ΔGLN3a          KLKGSDNTFECQFDDESATVVDFLRRWIAFCQSIISTSPQ.
● ΔGLN4a          KLKGSDNTFECQFDDESATVVDFLRRWIAFCQSIISTSPQ.
● ΔGLN6-2         KLKGSDNTFECQFDDESATVVDFLRRWIAFCQSIISTSPQ.
● ΔGLN7-2         KLKGSDNTFECQFDDESATVVDFLRRWIAFCQSIISTSPQ.
● ΔGLN3-2         KLKGSDNTFECQFDDESATVVDFLRRWIAFCQSIISTSPQ.
● ΔGLN6           KLKGSDNTFECQFDDESATVVDFLRRWIAFCQSIISTSPQ.
● ΔGLN2a          KLKGSDNTFECQFDDESATVVDFLRRWIAFCQSIISTSPQ.
● ΔGLN4-2         KLKGSDNTFECQFDDESATVVDFLRRWIAFCQSIISTSPQ.
● ΔGLN9b          KLKGSDNTFECQFDDESATVVDFLRRWIAFCQSIISTSPQ.
  ΔGLN15-2        KLKGSDNTFECQFDDESATVVDFLRRWIAFCQSIISTSPQ.
● 7-1  CT         KLKGSDNTFECQFDDESATVVDFLRRWIAFCQSIISTSPQ.
● 10-1 CT         KLKGSDNTFECQFDDESATVVDFLRRWIAFCQSIISTSPQ.
  ΔHIII 17
  ΔHIII 19        qLnrlaahppfaswrnSeeeartdrpsqqlrslngewrSdavfspyasvryftphmvhsqynll.
  ΔHIII           qLnrlaahppfaswrnSeeeartdrpsqqlrslngewrSdavfspyasvryftphmvhsqynll.
  ΔXho 5
  ΔXho 7
  ΔXho 8
  Mlu12-1
  ΔMlu 22
  Bam-2           ILllStsyvdgslsvslssVllrsnlve.
  Bam-5           rricyccrlpt.
  ΔBam F1         lgtGrrfTts.
  ΔBam 12         rawawpsl.
  2-1  CT         Kr.
  14-2 CT         KLKGSgqhFrmsvrrrly.
  ΔSal D2         KLKGSDNTFECQFDDESATede.
  ΔSnaBI 3        KLKGSDNTFECQFDDESATVVDFLne.
  ΔSnaBI 2        KLKGSDNTFECQFDDESATVVDFLnesLAlavvlqrrdwenpgvtqlnrlaahppfasdrns.......
● ΔScaI 7         KLKGSDNTFECQFDDESATVVDFLRRWIAFCQSIISrkasygwhdskrimqcchnhe.
● ΔScaI 12        KLKGSDNTFECQFDDESATVVDFLRRWIAFCQSIIrllrsnlde.
● ΔScaI 5         KLKGSDNTFECQFDDESATVVDFLRRWIAFCQ    TSPQ.
● mIL2            KLKGSDNTFECQFDDESATVVDFLRRWIAFCQSIISTSPQ.
                  110      120      130      140
```

FIGURE 2

(C)